# FASCICULE DE BREVET EUROPEEN

(11) **EP 4 304 534 B1**
(45) Date de publication et mention de la délivrance du brevet: **18.06.2025**
(21) Numéro de dépôt: 22709332.5
(22) Date de dépôt: 08.03.2022
(51) Int. Cl.: A61F 5/02, A61F 5/03

(54) **DISPOSITIF D'AIDE A L'AMELIORATION DE LA POSTURE AVEC UN NOUVEAU SYSTÈME DE MAINTIEN**
HALTUNGSVERBESSERUNGSHILFE MIT NEUARTIGEM HALTESYSTEM
POSTURE IMPROVEMENT AID WITH NOVEL HOLDING SYSTEM

(30) Priorité: 11.03.2021 FR 2102405
(43) Date de publication de la demande: 17.01.2024
(73) Titulaire: Percko, 75010 Paris (FR)
(72) Inventeur: PERRAUDEAU, Quentin, 75010 Paris (FR); UCKO, Alexis, 75009 Paris (FR)
(74) Mandataire: Cabinet Camus Lebkiri
(86) Numéro de dépôt international: PCT/EP2022/055809
(87) Numéro de publication internationale: WO 2022/189388

(56) Documents cités:
- DE-U1- 202006 004 993
- JP-A- S61 239 002

## Description

### DOMAINE TECHNIQUE DE L'INVENTION

Le domaine technique de l'invention est celui des dispositifs d'aide pour améliorer la posture d'un utilisateur et notamment la correction d'une mauvaise posture et l'optimisation de la respiration.

La présente invention concerne en particulier le maintien en position de ces types de dispositifs.

### ARRIERE-PLAN TECHNOLOGIQUE DE L'INVENTION

Aujourd'hui, de nombreuses personnes souffrent de maux de dos qui sont dus notamment à une mauvaise posture aussi bien en position statique que dynamique.

Il existe des solutions permettant d'adopter et de maintenir une bonne posture sur la partie inférieure du dos d'un utilisateur tout en laissant l'utilisateur respirer correctement et être libre de ses mouvements. Mais pour être efficaces, ces dispositifs doivent être bien positionnés et rester en place pendant leur utilisation, faute de quoi ils peuvent être contre productifs. Ainsi le brevet WO 2016 198 358 décrit un tel dispositif de maintien attaché à la ceinture afin de rester en place et de garder une zone de stimulation au bon endroit. Ce dispositif est particulièrement adapté aux personnes âgées et aux femmes à forte poitrine qui peuvent l'enfiler plus facilement qu'un teeshirt près du corps.

Les personnels hospitaliers sont amenés à solliciter leur dos, notamment pour soulever des patients et ces dispositifs sont particulièrement adaptés. Cependant, ils portent une tenue qui comprend un pantalon maintenu à la taille par un élastique et les infirmières n'ont pas toujours de ceinture, notamment lorsqu'elle porte une blouse, ce qui fait que ces dispositifs ne tiennent pas toujours en place de façon satisfaisante.

On connait aussi des dispositifs adaptés pour les femmes enceintes, mais ces dispositifs sont maintenus en place par des sangles passant entre les jambes, ce qui n'est pas confortable, ni pratique car il peut être nécessaire de les défaire dans certaines situations. DE 20 2006 004993 divulgue un dispositif d'aide pour améliorer la posture d'un utilisateur suivant le préambule de la revendication 1.

### RESUME DE L'INVENTION

L'invention offre une solution aux problèmes évoqués précédemment, en permettant de maintenir en place les dispositifs de maintien malgré des mouvements amples et répétés et sans nécessiter de ceinture.

L'invention concerne un dispositif d'aide pour améliorer la posture d'un utilisateur comportant :
- deux premiers tenseurs latéraux comprenant chacun une première extrémité et une seconde extrémité, les premières extrémités de chaque premier tenseur latéral se rejoignant en une zone de stimulation,
- un support de fixation comprenant une partie arrière avec une première extrémité fixée à la zone de stimulation, et deux parties latérales fermées l'une avec l'autre par un moyen de serrage, il est caractérisé ne ce que la partie arrière comprend une deuxième extrémité qui se prolonge en dessous des fesses de l'utilisateur.

Grace au galbe des fesses, la partie inférieure du dispositif est maintenue en place même en mouvement. Les deux parties latérales permettent la maintien du dispositif lorsqu'elles sont fermées l'une avec l'autre, sans gène pour l'utilisateur.

La coopération du support de fixation avec les tenseurs latéraux permet au dispositif d'agir sur une zone spécifique du dos appelée zone de stimulation. La zone de stimulation, généralement positionnée entre la troisième vertèbre lombaire et la cinquième vertèbre lombaire de l'utilisateur, permet, en cas d'effacement de la courbure lombaire, une stimulation optimale du dos d'un utilisateur. En effet, un effacement de la courbure lombaire consiste, par exemple, à maintenir un dos arrondi qui entraine une déformation de la lordose lombaire physiologique. La lordose lombaire étant généralement localisée entre la troisième vertèbre lombaire et la cinquième vertèbre lombaire, une mise en tension de la zone de stimulation permet de stimuler directement la zone dont la courbure physiologique doit être rétablie. Ainsi, une utilisation régulière d'un tel dispositif permet à un utilisateur de combattre et de prévenir d'une mauvaise posture pouvant être à l'origine de maux de dos. En outre, le dispositif ne bloque pas l'ouverture du thorax de l'utilisateur, il peut ainsi respirer correctement tout en restant libre de ses mouvements.

Avantageusement, la partie arrière comprend un tenseur destiné à être positionné sous les fesses de l'utilisateur. Ce tenseur permet de garantir un meilleur maintien et que le support de fixation restera en place quelque soient les mouvements de l'utilisateur.

Avantageusement, le moyen de serrage est réglable. L'utilisateur peut ainsi régler la tension du maintien en fonction de sa morphologie. Le moyen de serrage peut être, par exemple, formés par un système de scratch, un nœud, une boucle ou un système de crochets.

Avantageusement, le dispositif comporte des moyens d'ouverture du thorax comprenant une partie avant et une partie arrière fixées à la seconde extrémité de chaque premier tenseur latéral, la jonction entre la partie avant et la partie arrière étant construite et agencé pour se positionner sur les épaules d'un utilisateur. Cela permet à l'utilisateur de maintenir son dos dans une bonne position tout en ne gênant pas sa respiration.

Avantageusement, la partie avant et la partie arrière des moyens d'ouverture du thorax forment des bretelles. Par « bretelle », on entend une bande de matière, pouvant être extensible, destinée à être positionnée verticalement de part et d'autre de l'épaule d'un utilisateur, une partie de ladite bretelle passant au-dessus de l'épaule de sorte à l'entourer.

Avantageusement le dispositif comporte deux tenseurs axiaux comprenant chacun une première extrémité fixée à la zone de stimulation et une seconde extrémité fixée aux moyens d'ouverture du thorax. Ces tenseurs permettent d'amplifier l'ouverture du thorax.

Avantageusement, le dispositif comprend deux seconds tenseurs latéraux comprenant une première extrémité et une seconde extrémité, les premières extrémités de chaque second tenseur latéral se rejoignant dans la zone de stimulation, les secondes extrémités de chaque second tenseur latéral sont fixées à la partie avant du support de fixation. On a ainsi un excellent maintien.

Avantageusement, le dispositif est réalisé avec un tissu respirant. Ceci permet notamment de ne pas transpirer notamment quand il fait chaud.

L'invention et ses différentes applications seront mieux comprises à la lecture de la description qui suit et à l'examen des figures qui l'accompagnent.

### BREVE DESCRIPTION DES FIGURES

Les figures sont présentées à titre indicatif et nullement limitatif de l'invention.
[Fig. 1] est une vue de dos du dispositif selon l'invention ;
[Fig. 2] est une vue de devant du dispositif de la figure 1 ;
[Fig. 3] montre une bretelle du dispositif selon l'invention
[Fig. 4] montre le dos d'un dispositif selon l'invention,
[Fig. 5] est une vue de face du dispositif de la figure 4 avec un autre système de fermeture.

### DESCRIPTION DETAILLEE

Sauf précision contraire, un même élément apparaissant sur des figures différentes présente une référence unique.

Dans la présente description, on considèrera que l'arrière correspond à la partie du dispositif placée dans le dos de l'utilisateur et l'avant à sa face avant. Le haut correspond à la partie du dispositif mise sur les épaules de l'utilisateur et le bas à la partie située en dessous de la taille.

Par « tenseur », on entend une bande de matière, pouvant être extensible, destinée à être positionnée verticalement ou obliquement sur une partie du corps d'un utilisateur.

Le dispositif est symétrique, une même numérisation sera utilisée dans la suite de la description pour désigner les tenseurs disposés symétriquement par rapport à la colonne vertébrale de l'utilisateur.

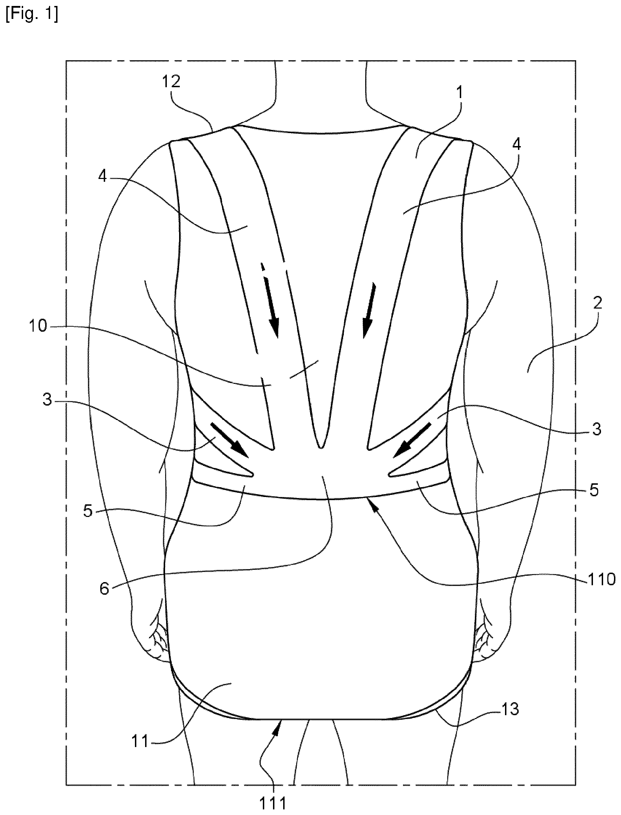

Le dispositif d'aide 1 pour améliorer la posture d'un utilisateur 2 illustré figure 1, comprend des bretelles 12, quatre tenseurs latéraux 3 et 5, et deux tenseurs axiaux 4. Les tenseurs axiaux 4 sont disposés de part et d'autre de la colonne vertébrale. Les tenseurs latéraux 3 et 5 sont placés par paire de chaque côté de la colonne vertébrale, un tenseur 5 sensiblement horizontalement et l'autre tenseur 3 incliné vers le haut. Tous ces tenseurs convergent vers une zone de stimulation 6.

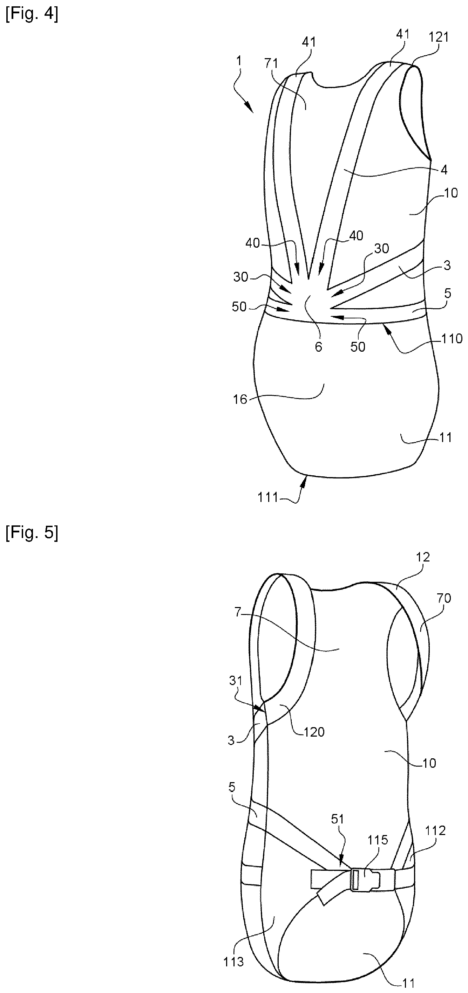

Les tenseurs latéraux 3 inclinés ont une première extrémité 30 et une deuxième extrémité 31, les tenseurs latéraux 5 horizontaux ont une première extrémité 50 et une deuxième extrémité 51 et les tenseurs axiaux 4 ont une première extrémité 40 et une deuxième extrémité 41. Les premières extrémités 30, 40 et 50 se rejoignent toutes dans la zone de simulation 6 (cf. figure 4).

Ces tenseurs sont fixés sur un tissu 10, de préférence élastique, qui se prolonge dans sa partie basse par un support de fixation 11. Ce support de fixation 11 a une première extrémité 110 fixée à la zone de stimulation 6 et une deuxième extrémité 111 se prolongeant en dessous des fesses de l'utilisateur.

La deuxième extrémité 111 qui passe sous les fesses de l'utilisateur peut comprendre un tenseur de maintien 13 qui évite que le support de fixation bouge en cas de mouvement brusque ou si l'utilisateur s'assoie rudement.

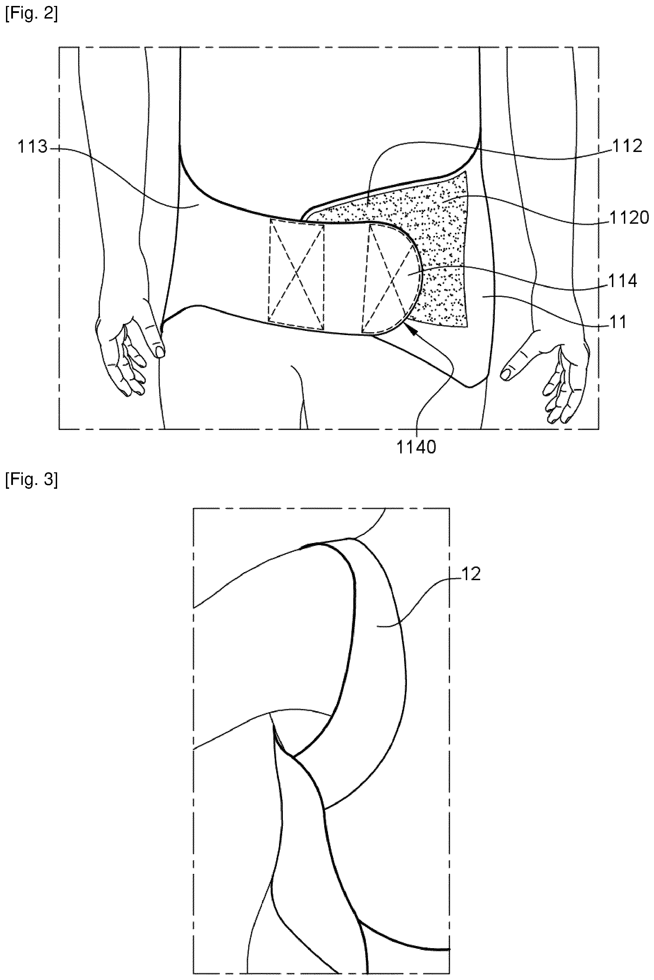

On peut voir figure 2 que le support de fixation 11 comprend une partie arrière 116 et deux parties latérales 112 et 113 se rejoignant à l'avant pour entourer le bassin et les fesses de l'utilisateur et sont fixées l'une à l'autre par une fermeture auto-agrippante 114 de type Velcro^{®}, ce qui permet un réglage en fonction de la corpulence de l'utilisateur et de la pression qu'il recherche. Dans cet exemple la partie latérale 112 présente une surface 1120 adaptée à recevoir la partie agrippante 1140 de la fermeture 114.

Comme illustré figure 5, les tenseurs latéraux 5 horizontaux ont leur deuxième extrémité 51 reliée à l'avant jusqu'à la fermeture qui est ici une boucle réglable 115.

Les deux tenseurs latéraux 3 inclinés ont leur deuxième extrémité 31 reliée à la partie base 120 de la bretelle 12 et les tenseurs axiaux 4 ont leur deuxième extrémité 41 reliée à la partie haute 121 de la bretelle 12.

## Revendications

1. Dispositif (1) d'aide pour améliorer la posture d'un utilisateur (2) comportant :
- deux premiers tenseurs latéraux (3) comprenant une première extrémité (30) et une seconde extrémité (31), les premières extrémités (30) du chaque premier tenseur latéral se rejoignant en une zone de stimulation (6),
- un support de fixation (11) comprenant une partie arrière (116) avec une première extrémité (110) fixée à la zone de stimulation (6), et deux parties latérales (112, 113) fermées l'une avec l'autre par un moyen de serrage (114, 115),
caractérisé ne ce que la partie arrière (116) comprend une deuxième extrémité (111) qui se prolonge en dessous des fesses de l'utilisateur (2).

2. Dispositif (1) selon la revendication 1, **caractérisé en ce que** la partie arrière (116) comprend un tenseur (13) destiné à être positionné sous les fesses de l'utilisateur.

3. Dispositif (1) selon l'une quelconque des revendications précédentes, **caractérisé en ce que** le moyen de serrage (114, 115) est réglable.

4. Dispositif (1) selon l'une quelconque des revendications précédentes, **caractérisé en ce qu'**il comporte des moyens d'ouverture du thorax (7) comprenant une partie avant (70) et une partie arrière (71) fixées à la seconde extrémité (31) du chaque premier tenseur latéral (3), la jonction entre la partie avant (70) et la partie arrière (71) étant construite et agencé pour se positionner sur les épaules d'un utilisateur (2).

5. Dispositif (1) selon la revendication précédente, **caractérisé en ce que** la partie avant (70) et la partie arrière (71) des moyens d'ouverture du thorax (7) forment des bretelles (12).

6. Dispositif (1) selon l'une quelconque des revendications 4 ou 5 **caractérisé en ce qu'**il comporte deux tenseurs axiaux (4) comprenant une première extrémité (40) fixée à la zone de stimulation (6) et une seconde extrémité (41) fixée aux moyens d'ouverture du thorax (7).

7. Dispositif (1) selon l'une quelconque des revendications précédentes **caractérisé en ce qu'**il comprend deux seconds tenseurs latéraux (5) comprenant une première extrémité (50) et une seconde extrémité (51), chaque première extrémité (50) des seconds tenseurs latéraux (5) se rejoignant en la zone de stimulation (6), les secondes extrémités des seconds tenseurs latéraux (5) sont fixées aux parties latérales (112, 113) du support de fixation (11).

8. Dispositif (1) selon l'une quelconque des revendications précédentes **caractérisé en ce qu'**il est réalisé avec un tissu respirant.

## Patentansprüche

1. Hilfsvorrichtung (1) zur Verbesserung der Körperhaltung eines Benutzers (2), umfassend:
- zwei erste seitliche Spanner (3), die ein erstes Ende (30) und ein zweites Ende (31) umfassen, wobei die ersten Enden (30) jedes ersten seitlichen Spanners in einen Stimulationsbereich (6) zusammenlaufen,
- eine Befestigungshalterung (11), die einen hinteren Teil (116) mit einem ersten Ende (110), das an dem Stimulationsbereich (6) befestigt ist, und zwei Seitenteile (112, 113) umfasst, die durch ein Klemmmittel (114, 115) miteinander verschlossen sind,
**dadurch gekennzeichnet, dass** der hintere Teil (116) ein zweites Ende (111) umfasst, das sich unterhalb des Gesäßes des Benutzers (2) erstreckt.

2. Vorrichtung (1) nach Anspruch 1, **dadurch gekennzeichnet, dass** der hintere Teil (116) einen Spanner (13) umfasst, der dazu bestimmt ist, unter dem Gesäß des Benutzers positioniert zu werden.

3. Vorrichtung (1) nach einem der vorhergehenden Ansprüche, **dadurch gekennzeichnet, dass** das Klemmmittel (114, 115) einstellbar ist.

4. Vorrichtung (1) nach einem der vorhergehenden Ansprüche, **dadurch gekennzeichnet, dass** sie Mittel zum Öffnen des Brustkorbs (7) aufweist, die einen vorderen Teil (70) und einen hinteren Teil (71) umfassen, die am zweiten Ende (31) jedes ersten seitlichen Spanners (3) befestigt sind, wobei die Verbindung zwischen dem vorderen Teil (70) und dem hinteren Teil (71) so gestaltet und angeordnet ist, dass sie auf den Schultern eines Benutzers (2) positioniert werden kann.

5. Vorrichtung (1) nach dem vorhergehenden Anspruch, **dadurch gekennzeichnet, dass** der vordere Teil (70) und der hintere Teil (71) der Mittel zum Öffnen des Brustkorbs (7) Schultergurte (12) bilden.

6. Vorrichtung (1) nach einem der Ansprüche 4 oder 5, **dadurch gekennzeichnet, dass** sie zwei axiale Spanner (4) umfasst, die ein erstes Ende (40), das am Stimulationsbereich (6) befestigt ist, und ein zweites Ende (41), das an den Mitteln zum Öffnen des Brustkorbs (7) befestigt ist, umfassen.

7. Vorrichtung (1) nach einem der vorhergehenden Ansprüche, **dadurch gekennzeichnet, dass** sie zwei zweite seitliche Spanner (5) mit einem ersten Ende (50) und einem zweiten Ende (51) umfasst, wobei jedes erste Ende (50) der zweiten seitlichen Spanner (5) im Stimulationsbereich (6) zusammenläuft, die zweiten Enden der zweiten seitlichen Spanner (5) an den Seitenteilen (112, 113) der Befestigungshalterung (11) befestigt sind.

8. Vorrichtung (1) nach einem der vorhergehenden Ansprüche, **dadurch gekennzeichnet, dass** sie aus einem atmungsaktiven Stoff hergestellt ist.

## Claims

1. An aid device (1) to improve the posture of a user (2) including:
- first two lateral tensioners (3) comprising a first end (30) and a second end (31), the first ends (30) of each first lateral tensioner meeting in a stimulation zone (6),
- an attachment support (11) comprising a rear part (116) with a first end (110) attached to the stimulation zone (6), and two lateral parts (112, 113) closed together by a clamping means (114, 115),
**characterised in that** the rear part (116) includes a second end (111) which extends from the bottom of the user's (2) buttocks.

2. The device (1) according to claim 1, **characterised in that** the rear part (116) includes a tensioner (13) to be positioned under the user's buttocks.

3. The device (1) according to any of the preceding claims, **characterised in that** the clamping means (114, 115) is adjustable.

4. The device (1) according to any of the preceding claims, **characterised in that** it includes means for opening the thorax (7) comprising a front part (70) and a rear part (71) attached to the second end (31) of each first lateral tensioner (3), the junction between the front part (70) and the rear part (71) being constructed and arranged to be positioned on the shoulders of a user (2).

5. The device (1) according to the preceding claim, **characterised in that** the front part (70) and the rear part (71) of the means for opening the thorax (7) form shoulder straps (12).

6. The device (1) according to any of claims 4 or 5, **characterised in that** it includes two axial tensioners (4) comprising a first end (40) attached to the stimulation zone (6) and a second end (41) attached to the means (7) for opening the thorax.

7. The device (1) according to any of the preceding claims, **characterised in that** it comprises two second lateral tensioners (5) comprising a first end (50) and a second end (51), each first end (50) of the second lateral tensioners (5) meeting at the stimulation zone (6), the second ends of the second lateral tensioners (5) are attached to the lateral parts (112, 113) of the attachment support (11).

8. The device (1) according to any of the preceding claims, **characterised in that** it is made with a breathable fabric.
